Europäisches Patentamt

**European Patent Office**

Office européen des brevets

(19)

(11) Veröffentlichungsnummer: **0 028 202**
**B1**

(12) **EUROPÄISCHE PATENTSCHRIFT**

(45) Veröffentlichungstag der Patentschrift:
**22.02.84**

(51) Int. Cl.³: **A 61 B 5/04,** A 61 B 5/10

(21) Anmeldenummer: **80730067.8**

(22) Anmeldetag: **15.10.80**

(54) Amplitudenregeleinrichtung für elektromyographisch aufgenommene Signale.

(30) Priorität: **26.10.79 DE 2943781**

(43) Veröffentlichungstag der Anmeldung:
**06.05.81 Patentblatt 81/18**

(45) Bekanntmachung des Hinweises auf die Patenterteilung:
**22.02.84 Patentblatt 84/8**

(84) Benannte Vertragsstaaten:
**AT CH DE FR GB IT LI NL SE**

(56) Entgegenhaltungen:
**EP - A - 0 000 504**
**DE - A - 2 601 704**
**US - A - 3 945 374**

(73) Patentinhaber: **BIOTRONIK Mess- und Therapiegeräte GmbH & Co Ingenieurbüro Berlin, Sieversufer 8, D-1000 Berlin 47 (DE)**

(72) Erfinder: **Nagel, Joachim, Dipl.-Phys. Dr., Schobertweg 3A, D-8520 Erlangen (DE)**

(74) Vertreter: **Christiansen, Henning, Dipl.-Ing., Unter den Eichen 108a, D-1000 Berlin 45 (DE)**

Amplitudenregeleinrichtung für elektromyographisch aufgenommene Signale

Die Erfindung betrifft eine Einrichtung zur Verarbeitung eines elektromyographisch aufgenommenen Signals, welches elektrokardiographische Anteils enthält.

Eine derartige Einrichtung ist aus der US-A-3 945 374 bekannt. Bei dieser geht es jedoch darum, in dem aufgenommenen Signal die elektrokardiographischen Anteile zu unterdrükken.

Der Gegenstand der Erfindung bezieht sich dagegen auf eine Anordnung, welche den Ausgleich unterschiedlicher Ableitungsverhältnisse bei der Überwachung der Uterusaktivität zum Gegenstand hat.

Die Überwachung und Auswertung der Wehentätigkeit ist in der perinatalen Medizin wichtig zur Überwachung des Verlaufs der Schwangerschaft und zur Beurteilung des Zustands der Feten. Da das mit der Uteruskontraktion einhergehende elektrische Feld sich gegen der großen Ausdehnung und geringen Reizleitungsgeschwindigkeit der Uterusmuskulatur wesentlich von demjenigen der Herzaktionen unterscheidet und die Wellenform der extern abgeleiteten Spannungskurve, des Elektromyogramms, eher den Verlauf eines stochastischen Signals aufweist, sind zur Registrierung der Wehenkurven besondere Maßnahmen erforderlich.

Bei einer bekannten Vorrichtung (EP-A1-0 000 504) wird bei einer möglichen Ausführungsform die elektromyographische Registrierung durch Gleichrichtern und anschließendes Tiefpaßfiltern der abgeleiteten Signale durchgeführt.

Unabhängig von der Art der Verarbeitung der ein Maß für die Wehentätigkeit bildenden Signale werden diese durch die Wehen selbst störend beeinflußt. Während der Wehen verändert sich nämlich die Position der Elektroden relativ zu den die Signalquellen bildenden Muskelfasern. Durch den Wechsel der Übertragungswege sind Variationen der Amplitude des Ausgangssignals bedingt, die in ihrem Zusammenhang mit der Wehentätigkeit nicht ohne weiteres vorhersagbar sind. Die Abhängigkeit der Amplituden der an der Körperoberfläche meßbaren elektrischen Aktionspotentiale vom Übertragungsweg im Körperinneren, die beispielsweise auch durch Lageänderungen der Schwangeren bedingt sein können, führen bei der Registrierung der Wehenkurve zu einer Verschiebung der Nullinie.

Die Benutzung einer einfachen Amplitudenregelung würde dabei keine Abhilfe schaffen, da hierdurch lediglich eine »Einebnung« der Amplitudenschwankungen, bezogen auf längere Zeiträume, erreicht werden könnte, was aber angesichts der damit verbundenen großen Zeitkonstanten und der Verfälschungen des Nutzsignals nicht erstrebenswert ist.

Der Erfindung liegt die Aufgabe zugrunde, eine Vorrichtung der obengenannten Gattung zu schaffen, die es ermöglicht, die ausgegebenen Wehenkurven von Signalschwankungen zu befreien, die nicht unmittelbar durch mit der Muskelaktivität einhergehenden wechselnden Aktionspotentialen, sondern durch sekundäre Einflüsse hervorgerufen werden.

Diese Aufgabe wird dadurch gelöst, daß zur Registrierung der Uterusaktivität Mittel vorgesehen sind, um als Maß für intrauterine Druckänderungen die mit Muskelaktivitäten einhergehenden elektrischen Felder im abdominalen Bereich elektromyographisch zu erfassen, wobei die Amplitudenwerte des maternellen Elektrokardiogramms als Normierungsgröße für die Amplitudenregelung des elektromyographisch abgeleiteten Signals dienen.

Die Erfindung geht aus von der Erkenntnis, daß die das maternelle EKG bildenden Signale in ihrer Ausbreitung innerhalb des Körpers in erster Näherung denselben Veränderungen unterworfen sind wie die für die Uterusaktivität charakteristischen Signale. Sowohl Änderungen hinsichtlich des Ortes der Ableitung als auch solche, die den Ausbreitungsweg der Signale betreffen, und somit die abzuleitenden Signale beeinflussen, wirken sich auf beide Signale nahezu übereinstimmend aus, so daß das maternelle EKG, und dabei vorzugsweise die R-Zacke, eine Art Eichsignal für das EMG bilden kann. Dabei braucht die genaue Variation der Signalübertragungsstrecke im Körperinneren nicht bekannt zu sein. Die unterschiedliche Position des mütterlichen Herzens im Vergleich zum Ort der Herkunft der für die Wehenkurve verantwortlichen Signale spielt überraschenderweise nur eine untergeordnete Rolle.

Besonders vorteilhaft ist bei der erfindungsgemäßen Einrichtung, daß sie keine zusätzliche Aufmerksamkeit bei der Bedienung erfordert und wegen der relativ großen Amplitude der maternellen Herzsignale und der sich daraus ergebenden Sicheren Regelwirkung eine sichere und unkomplizierte Betriebsweise ermöglicht.

Ein Ausführungsbeispiel der Erfindung ist in den Figuren dargestellt und wird nachfolgend beschrieben. Es zeigt

Fig. 1 ein Blockschaltbild, welches Aufschluß gibt über die Anordnung der erfindungsgemäßen Amplitudenregelung innerhalb einer Vorrichtung zur Registrierung der Uterustätigkeit,

Fig. 2 eine detaillierte Schaltung der Amplitudenregelung gemäß Fig. 1 sowie

Fig. 3a und 3b eine zeitabhängige Darstellung des Amplitudenverlaufes der mit den Uteruskontraktionen einhergehenden Signalen mit bzw. ohne Amplitudenregelung.

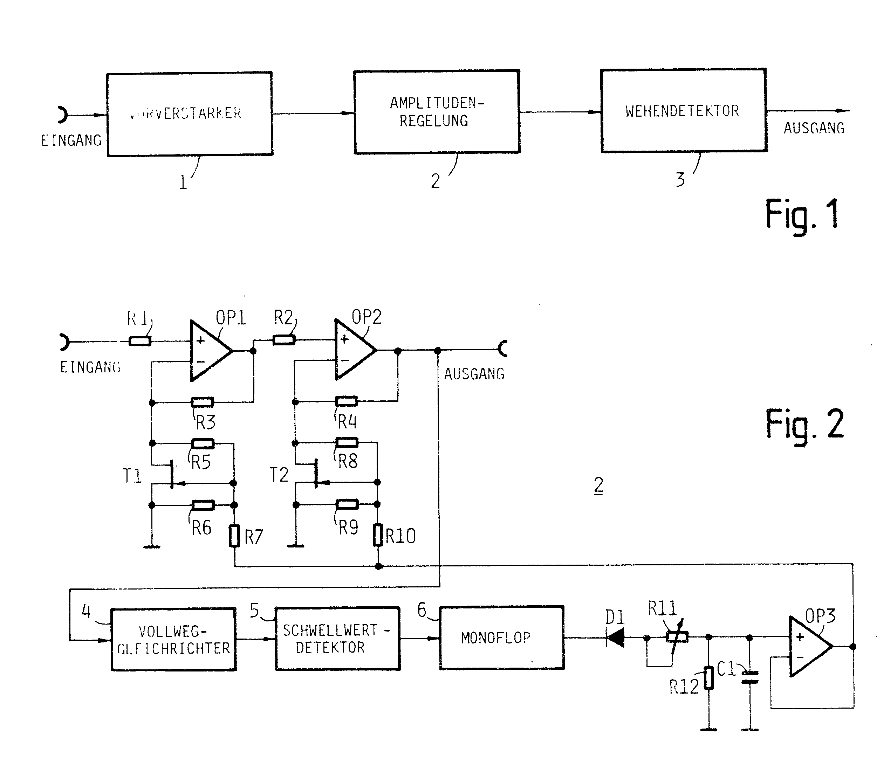

Bei dem in Fig. 1 dargestellten Ausführungsbeispiel wird das über Elektroden abdominal abgeleitete elektromyographische Signal einem Vorverstärker 1 zugeführt, wo es in seiner Amplitude soweit hinaufgesetzt wird, daß in den nachfolgenden Stufen die eigentliche Signalverarbeitung vorgenommen werden kann.

Das Ausgangssignal des Vorverstärkers 1 gelangt zu einer Amplitudenregelschaltung 2, die nachfolgend näher dargestellt ist. Das in seiner Amplitude beeinflußte Signal wird einem Wehendetektor 3 zugeführt, in dem ein Ausgangssignal erzeugt wird, welches in seinem zeitlichen Verlauf eine die Uteruskontraktionen repräsentierende Amplitude aufweist, wie es im einzelnen in der EP-A1-0 000 504 näher beschrieben ist. Das Ausgangssignal kann auf beliebige Weise visuell dargestellt oder in anderer Weise registriert werden.

Die in Fig. 2 dargestellte detaillierte Schaltung der Ausführungsform der Amplitudenregelschaltung 2 in Fig. 1 ermöglicht eine Verstärkungsregelung durch die im Signal selbst auftretenden Spitzenwerte. Die Regelung erfolgt dabei in dem Sinne, die möglichst die Proportionalität des Ausgangssignals, bezogen auf die verursachenden Ereignisse — d. h. die Uteruskontraktionen —, hergestellt wird.

Da das maternelle QRS-Komplexe — und darin die R-Zacken — die Signale mit der größten Amplitude innerhalb des Eingangssignalgemisches darstellen, ist es durch Diskrimination bezüglich der auftretenden absoluten Signalspitzen in besonders einfacher Weise möglich, eine Trennung der das »Eichsignal« bildenden Signalanteile von dem übrigen zu verarbeitenden Signalgemisch zu erreichen.

Die Operationsverstärker OP1 und OP2 entsprechen üblichen Schaltungen, wobei die Widerstände R1 und R2 die Eingangswiderstände darstellen und das Verstärkungsverhältnis jeweils über den durch einen Widerstand R3 und einen Feldeffekttransistor T1 bzw. einen Widerstand R4 und einen Feldeffekttransistor T2 gebildeten Spannungsteiler beeinflußt wird. Als Spannungsteiler für die Gate-Elektroden T1 bzw. T2 dienen Widerstände R5 und R6 bzw. R8 und R9, wobei die Transistoren T1 und T2 ihren Widerstand innerhalb eines vorgesehen Arbeitsbereichs in Abhängigkeit von der am Ausgang eines Operationsverstärkers OP3 erscheinenden Spannung verändern, die ihren Eingängen über Widerstände R7 und R10 zugeführt wird. Das Ausgangssignal des Operationsverstärkers OP2, das gleichzeitig das Ausgangssignal der Amplitudenregelschaltung 2 in Fig. 1 bildet, wird einem Vollweg-Gleichrichter 4 zugeführt, durch den die Signalspannungsspitzen für die nachfolgende Bearbeitung die gleiche Polarität erhalten.

Der Vollweg-Gleichrichter 4 kann ebenfalls durch Operationsverstärker-Schaltungen gebildet werden, wie sie mit der entsprechenden Beschaltung aus der einschlägigen Literatur bekannt sind. Das Ausgangssignal des Vollweg-Gleichrichters 4 gelangt zu einem Schwellwert-Detektor 5 (Schmitt-Trigger), von dem aus die einen vorgegebenen Pegel überschreitenden Signale zu einem Monoflop 6 gelangen, welches durch diese Signale getriggert wird und daraufhin jeweils Ausgangsimpulse vorgegebener Breite abgibt.

Die negativen Ausgangsimpulse vom Monoflop 6 werden zu einer Diode D1 und einem veränderlichen Widerstand R11 geführt, wodurch ein Kondensator C1 auf negatives Potential aufgeladen wird. Die Spannung am Kondensator C1 bildet daher ein Maß für die Spitzenwerte für die am Ausgang des Operationsverstärkers OP2 erscheinenden Signale. Bei der vorliegenden Anwendung handelt es sich dabei um die Herzsignale des maternellen Elektrokardiogramms.

Die Regelung erfolgt dabei so, daß bei vergrößerter Spannung am Kondensator C1 der als Impedanzwandler geschaltete Operationsverstärker OP3 über die Transistoren T1 und T2 der Verstärkungsfaktor der Operationsverstärkerschaltungen OP1 und OP2 herabgesetzt wird, so daß sich daraufhin auch die Amplitude der den Kondensator C1 aufladenen Impulse vermindert bis ein Gleichgewichtszustand erreicht ist.

Ist die Amplitude des maternellen EKGs hingegen zu klein, um den Gleichgewichtszustand aufrecht zu erhalten, wobei keine Impulse am Ausgang des Monoflops 3 erscheinen, wird der Kondensator C1 über den Widerstand R12 entladen. Der Verstärkungsfaktor der Operationsverstärker OP1 und OP2 wird solange heraufgesetzt, bis die Amplitude der R-Zacken des maternellen Elektrokardiogramms im elektromyographisch aufgenommenen Signal wieder ihren Sollwert erreicht hat.

Die Regelung ist ersichtlicherweise nichtlinear, da eine Herabsetzung des Verstärkungsfaktors der Operationsverstärker OP1 und 2 erst dann hervorgerufen wird, wenn vom Ausgang des Monoflops 6 Impulse abgegeben werden. In ihrem Arbeitsbereich ist der Widerstand R11 im Hinblick auf die durch den Widerstand R12 und den Kondensator C1 gebildete Zeitkonstante so einzustellen, daß bei einer Verringerung der Amplituden der maternellen QRS-Komplexe der Verstärkungsfaktor ausreichend schnell heraufgesetzt wird, um die notwendige Anpassung an die Veränderungen der Signalausbreitung zu gewährleisten. Bleiben die Amplituden der QRS-komplexe näherungsweise konstant, so wird die Verstärkung der Operationsverstärker OP1 und 2 mit der Entladung des Kondensators C1 langsam heraufgesetzt, bis ein Impuls am Ausgang des Monoflops 6 erscheint, worauf sich die Spannung am Kondensator C1 wieder um einen Differenzbetrag erhöht, so daß die Verstärkung entsprechend herabgesetzt wird. Hierdurch treten periodische Schwankungen des Verstärkungsfaktors auf, die die Meßergebnisse jedoch nicht beeinträchtigen, da die maternelle Herzfrequenz groß ist im Vergleich zur Frequenz der Wehenkurven und somit die Schwankungen nicht sichtbar werden.

Durch diese Regelung, welche mit einer verhältnismäßig steilen Regelkennlinie das Ausgangssignal mit großer Einstellgeschwindigkeit

beeinflußt, wobei die genannten R-Zacken ein Bezugs- oder Eichsignal bilden, wird das für die Uterusaktivität repräsentative Signal unabhängig von den bei der Ausbreitung im Körper und der Abnahme der Signale entstehenden Veränderungen so eingestellt, daß die registrierte Amplitude ein zuverlässiges Maß für die Intensität für die Uteruskontraktion darstellt.

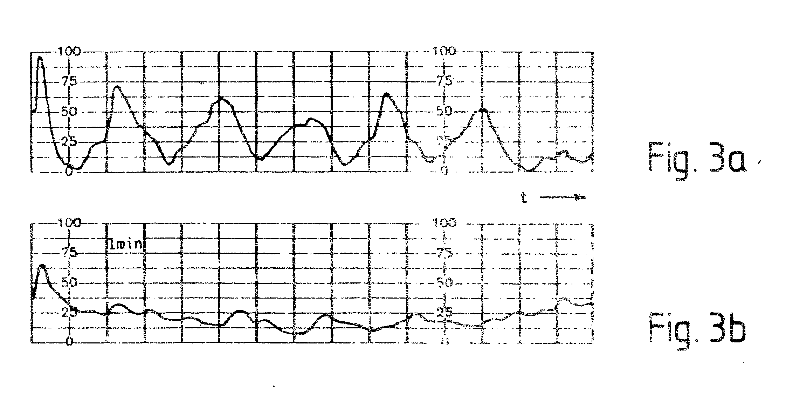

Die in den Fig. 3a und 3b wiedergegebenen Wehenkurven (Fig. 3a mit Amplitudenregelung und Fig. 3b ohne Amplitudenregelung) zeigen deutlich, welche Verbesserung durch die Erfindung in der Praxis erzielt werden konnte, so daß dem behandelnden Arzt eine Darstellung an die Hand gegeben werden kann, welche ohne mühsame Interpretationen direkten und zuverlässigen Aufschluß über die Wehentätigkeit gibt.

**Patentansprüche**

1. Einrichtung zur Verarbeitung eines elektromyographisch aufgenommenen Signals, welches elektrokardiographische Anteile enthält, dadurch gekennzeichnet, daß zur Registrierung der Uterusaktivität Mittel vorgesehen sind, um als Maß für intrauterine Druckänderungen die mit Muskelaktivitäten einhergehenden elektrischen Felder im abdominalen Bereich elektromyographisch zu erfassen, wobei die Amplitudenwerte des maternellen Elektrokardiogramms als Normierungsgröße für die Amplitudenregelung des elektromyographisch abgeleiteten Signals dienen.

2. Vorrichtung nach Anspruch 1, dadurch gekennzeichnet, daß die Amplitudenwerte des maternellen Elektrokardiogramms aus den Amplituden der Spitzenwerte (R-Zacken) bestehen.

3. Vorrichtung nach einem der vorangehenden Ansprüche, dadurch gekennzeichnet, daß die Amplitudenregelung durch Veränderung des Verstärkungsfaktors eines Verstärkers (OP1 und 2) für das elektromyographisch abgeleitete Signal erfolgt.

4. Vorrichtung nach Anspruch 3, dadurch gekennzeichnet, daß die Regelung nichtlinearer in der Weise erfolgt, daß eine die Verstärkung herabsetzende Größe erzeugt wird, wenn die Amplitude der Spitzenwerte des maternellen Elektrokardiogramms eine vorgegebene Schwelle überschreitet.

5. Vorrichtung nach Anspruch 4, dadurch gekennzeichnet, daß ein Schwellwertdetektor (5) vorgesehen ist, dessen Ausgangssignale über eine Zeitkonstante (R12, C1) ein Herabsetzen des Verstärkungsfaktors des Verstärkers (OP1 und 2) bewirken, wenn das dem Schwellwertdetektor (5) zugeführte elektromyographisch abgeleitete Signal eine vorgegebene Schwelle überschreitet.

**Patent Claims**

1. Arrangement for processing of an elektromyographically received signal which contains electrocardiographic components, characterised in that for the registration of uterus activity means are provided to detect elektromyographically the electrical fields in the abdominal region which accompany muscle activity, the amplitude values of the maternal electrocardiogram serving as standardization magnitude for the control of the amplitude of the electromyographically derived signal.

2. Arrangement according to claim 1, characterised in that the amplitude values of the maternal electrocardiogram consist of the amplitudes of the peak values (R-spike).

3. Arrangement according to any one of the preceding claims characterised in that the amplitude control is achieved by adjustment of the amplification factor of an amplifier (OP1 and 2) for the electromyographically derived signal.

4. Arrangement according to claim 3, characterized in that the control occurs in a non-linear manner so that a magnitude is produced which reduces the amplification when the amplitude of the peak values of the maternal electrocardiogram exceeds a predetermined threshold.

5. Arrangement according to claim 4, characterised in that there is provided a threshold value detector (5), the output signals of which via a time constant (R12, C1) cause a reduction of the amplification factor of the amplifier (OP1 and 2) when the elektromyographically derived signal supplied to the threshold value detector (5) exceeds a predetermined threshold.

**Revendications**

1. Dispositif pour traiter un signal recueilli par voie électromyographique et qui contient des fractions électrocardiographiques, caractérisé en ce que, dans le but d'enregistrer l'activité utérine, des moyens sont prévus pour détecter, dans la région abdominale, par voie électromyographique et comme mesure de variations de pression intrautérines, les champs électriques inhérents aux activités musculaires, les valeurs d'amplitude de l'électrocardiogramme maternel servant de grandeur de cadrage de la régulation d'amplitude du signal obtenu électromyographiquement.

2. Dispositif selon la revendication 1, caractérisé en ce que les valeurs d'amplitude de l'électrocardiogramme maternel sont constituées par les amplitudes des valeurs de crête (dents R).

3. Dispositif selon une des revendications précédentes, caractérisé en ce que la régulation d'amplitude s'effectue par variation du gain d'un amplificateur (OP1 et 2) pour le signal obtenu électrographiquement.

4. Dispositif selon la revendication 3, caractérisé en ce que la régulation s'effectue de façon non linéaire en se sens qu'une grandeur réduisant le gain est produite lorsque l'amplitude des valeurs de crête de l'électrocardiogramme dépasse un seuil préfixé.

5. Dispositif selon la revendication 4, caractérisé en ce qu'un détecteur de seuil (5) est prévu, dont les signaux de sortie produisent, à travers un dispositif à constante de temps (R12, C1), la réduction du gain de l'amplificateur (OP1 et 2) lorsque le signal obtenu électromyographiquement et appliqué au détecteur de valeur de seuil (5) dépasse un seuil préfixé.

Fig. 1

Fig. 2

0 028 202

Fig. 3a

Fig. 3b